(19) Europäisches Patentamt
European Patent Office
Office européen des brevets

(11) Veröffentlichungsnummer: **0 187 956**
**A2**

# (12) EUROPÄISCHE PATENTANMELDUNG

(21) Anmeldenummer: 85115779.2

(22) Anmeldetag: 11.12.85

(51) Int. Cl.[4]: **A 61 K 37/02**

(30) Priorität: 17.12.84 DE 3445932

(43) Veröffentlichungstag der Anmeldung:
23.07.86 Patentblatt 86/30

(84) Benannte Vertragsstaaten:
AT BE CH DE FR GB IT LI LU NL SE

(71) Anmelder: BOEHRINGER INGELHEIM KG

D-6507 Ingelheim am Rhein(DE)

(84) Benannte Vertragsstaaten:
BE CH DE FR IT LI LU NL SE AT

(71) Anmelder: Boehringer Ingelheim International G.m.b.H

D-6507 Ingelheim am Rhein(DE)

(84) Benannte Vertragsstaaten:
GB

(72) Erfinder: Brecht, Hans-Michael, Dr.
Turnerstrasse 12
D-6507 Ingelheim/Rhein(DE)

(72) Erfinder: Giachetti, Antonio, Prof. Dr.
Guerrini 3
Mailand(IT)

(72) Erfinder: Roos, Otto, Dr.
Elsheimer Strasse 36
D-6501 Schwabenheim(DE)

(72) Erfinder: Schnorrenberg, Gerd, Dr.
Ernst-Ludwig-Strasse 66a
D-6535 Gau-Algesheim(DE)

(72) Erfinder: Lösel, Walter, Dr.
Im Herzenacker 26
D-6535 Gau-Algesheim(DE)

## (54) Pharmazeutische Zusammensetzung zur Behandlung von Schleimhautdefekten des Magen-Darmtraktes.

(57) Verwendung von ACE-Hemmer und ähnlichen Verbindungen zur Herstellung eines Arzneimittels zur Behandlung von Schleimhautdefekten des Magen-Darmtraktes.

BEZEICHNUNG GEÄNDERT siehe Titelseite



Zahlreiche Angiotensin converting enzyme Hemmer (ACE-Hemmer) sind seit Jahren bekannt als Blutdrucksenker, deren Wirkung auf der Blockade des Renin-Angiotensin-Systems beruht. Besonders eingehend untersucht wurden das D-2-Methyl-3-mercaptopropanoyl-L-prolin, bekannt unter dem generic name Captopril sowie der 1-{N-[(S)-1-Carboxy-3-phenyl-propyl]-L-alanyl}-L-prolin-1'-ethylester (Enalapril).

Seit einigen Jahren sind insbesondere für Captopril weitere Wirkungen aufgefunden worden. So erwies es sich als effektiv in der Behandlung der rheumatoiden Arthritis (1), des Raynaud-Syndroms (2), von Depressionen (3), der Herzinsuffizienz (4) und bei Schmerzzuständen, z.B. Migräne (5), (6).

Überraschenderweise wurde gefunden, daß Verbindungen, die zum großen Teil als ACE-Hemmer bekannt sind, insbesondere solche, die sich von Aminosäuren oder Dipeptiden ableiten, eine starke mucoprotektive Wirkung auf den Gastrointestinaltrakt entfalten, die in vielen Fällen weit über die entsprechende Wirkung einer gleichen Dosis des bekannten Handelsproduktes Carbenoxolone hinausgeht.

Gegenstand der Erfindung sind daher pharmazeutische Zusammensetzungen mit mucoprotektiver Wirkung auf den Gastrointestinaltrakt, enthaltend als Wirkstoff eine oder mehrere Verbindungen der Struktur:

A-B-P I.

in der A für geradkettigen oder verzweigten Alkyl- beziehungsweise Acylrest, der substituiert sein kann durch SH, COOH oder COO-Alkyl und der gegebenenfalls unterbrochen sein kann, durch eine CO-Gruppe oder ein Schwefelatom; einen Phenylalkyl- beziehungsweise Phenylacylrest, wobei die Alkyl- beziehungsweise Acylkette geradkettigen oder verzweigt und ein- oder vielfältig substituiert sein kann, durch SH, S-Alkyl, S-Acyl, S-Alkylaminocarbonyl, COOH, COO-Alkyl oder $NO_2$ und gegebenenfalls unterbrochen sein kann, durch eine CO-Gruppe oder ein Schwefelatom, steht;

P für Carboxypyrrolidinyl oder Carboxypiperidinyl gegebenenfalls substituiert durch eine oder mehrere Hydroxy- oder Oxogruppe oder gegebenenfalls geminal substituiert durch Dialkoxy, Dithioalkoxy, Alkylendioxy oder Alkylendithio, und/oder gegebenenfalls substituiert durch/oder ankondensiert mit einem 5- oder 6-Ring-Heterocyclus wie Furan, Pyrrol, Thiophen, Benzofuran, Indol, Benzothiophen, Oxazol, Imidazol, Thiazol, Isoxazol, Pyrazol, Pyrrolidin, Tetrahydrofuran, Tetrahydrothiophen, Pyridin, Pyridazin, Chinolin, Isochinolin oder Piperidin, oder Thiazolidincarbonsäure steht;

und B für eine Einfachbindung, eine Aminosäure oder mehrere über Peptidbindung(en) miteinander verknüpften Aminosäuren, steht,

und falls das Molekül eine SH-Gruppe enthält, über eine -S-S-Bindung ein Doppelmolekül bilden kann;

oder ihre pharmazeutisch annehmbaren Salze, Ester oder Amide.

Der Begriff "Alkyl", "Alkylen" oder "Acyl" betrifft $C_1$-$C_8$-Radikale, die entweder allein stehen oder Bestandteil eines weiteren Substituenten sind. Die $C_1$-$C_3$-Alkyl, -Alkylen und -Acylradikale sind bevorzugt.

Ein geeigneter Wert für A ist beispielsweise die 3-Mercaptopropionyl, 3-Mercapto-2-methylpropionyl, 3-Acetylthio-2-methylpropionyl, 3-Mercaptobutyryl, 2-Methyl-4-ethoxy-carbonylpentanoyl, 3-Acetylthiobutyryl, 3-Trimethylacetylthiobutyryl, Hydroxycarbonylmethylthiopropionyl, 3-Phenyl-2-mercaptopropionyl, 3-Phenyl-2-methylmercaptopropionyl, 3-Phenyl-2-benzoylmercapto-propionyl, 3-Phenyl-2-trimethylacetylmercaptopropionyl, 3-Phenyl-2-ethylaminocarbonyl-mercaptopropionyl, 1-Ethoxycarbonyl-3-phenylpropyl, 1-Hydroxycarbonyl-3-phenylpropyl oder 3-(2-Nitro-1-phenylethylthio)-2-methylpropionyl-Gruppe.

Bevorzugte Werte für A sind

3-Acetylthio-2-methylpropionyl;
3-Mercapto-2-methylpropionyl;
3-Phenyl-2-mercaptopropionyl;
1-Ethoxy-3-phenylpropyl;
1-Hydroxycarbonyl-3-phenylpropyl; und
3-(2-Nitro-1-phenylethylthio)-2-methylpropionyl-Gruppe.

Bevorzugt ist B, eine Einfachbindung oder ein Aminosäurerest der sich von einer der Aminosäuren Glycin, Alanin, Serin oder Lysin ableitet.

Vorzugsweise steht P für 2-Carboxypyrrolidinyl, 4,5,6,7-tetrahydrothieno[3,2-c]piperidinyl-4-carbonsäure oder 4,5,6,7-tetrahydrothieno[2,3-c]piperidinyl-7-carbonsäure. Ein anderer geeigneter Wert für P ist 4-Keto.-2-carboxypyrrolidinyl, 4-Hydroxyprolin, 2-carboxypyrrolidinyl, 2-Carboxy-4-di($C_1$-$C_3$)alkylketal-pyrrolidinyl oder 2-Carboxy-4-dithio($C_1$-$C_3$)alkylketal-pyrrolidinyl (offen oder cyclisch), wie z.B. 2-Carboxy-4,4-ethylendioxy-pyrrolidinyl oder 2-Carboxy-4,4-ethylenedithio-pyrrolidinyl.

Außer den Aminosäuren mit freier Carboxylgruppe können auch die Salze, Ester und Amide dieser Aminosäuren für den erfindungsgemäßen Zweck eingesetzt werden.

Die Verbindung der Formel I kann basische oder saure Gruppen besitzen, z.B. Carboxylgruppe sowie Aminogruppe und können deshalb entweder als innere Salze, als Salze mit den üblichen pharmazeutisch verwendbaren Säuren, wie Salzsäure oder Maleinsäure oder als Salze mit pharmazeutisch verwendbaren Basen, z.B. Alkali- oder Erdalkalisalze wie Natrium, Kalium, Calcium oder Magnesium. Bei veresterten Aminosäuren sollte die Estergruppe leicht, z.B. enzymatisch, abspaltbar sein. Beispiele solcher Ester sind Alkyl-, Hydroxyalkyl- oder Acyloxyalkylester, wie Pivaloyloxymethyl, ferner der 2-Oxo-benzofuran-5-yl

oder der 1-Succinylimidoyl-1-ethylester. Amide der genannten Säuren leiten sich ab, beispielsweise von aliphatischen oder cyclischen Aminen, wie Alkyl- oder Dialkylaminen, Hydroxyalkylaminen, Cyclopentyl- oder Cyclohexylaminen, oder von Aminosäuren wie Valin, Prolin, Phenylalanin oder Glycin.

Die genannte Verbindungsklasse weist im allgemeinen mehrere Asymmetriezentren auf; die Verbindungen liegen daher als Diastereomere oder in Form ihrer Racemate beziehungsweise ihrer racemischen Gemische vor. Die Erfindung umfaßt sowohl die Verwendung der racemischen Gemische als auch die der einzelnen Diastereomeren. Bevorzugt werden diejenigen Enantiomeren verwendet, bei denen die asymmetrischen C-Atome in der L-Konfiguration vorliegen.

Die unter die oben angegebene Struktur fallenden Verbindungen sind bekannt beziehungsweise können nach bekannten Methoden erhalten werden, z.B. durch Verknüpfung des Restes A mit einer Aminosäure, beziehungsweise einem Dipeptid wie es beispielsweise angegeben ist in DBP 27 03 828, der Europa-Patentanmeldung Nr. 12 401 sowie den deutschen Offenlegungsschriften 30 11 239, 33 02 125, 33 02 126, 34 32 307, 34 36 569, 34 25 680 und den österreichischen Anmeldungen 5 A 1187/80 und 5 A 5688/80.

Als Verbindung der Struktur I sind hierbei besonders:

A. 1-(3-Mercapto-2-D-Methylpropanoyl)-L-prolin;

B. 1-{N-[(S)-1-Carboxy-3-phenylpropyl]-L-analyl}-L-prolin-1'-ethylester;

C. 1-(3-Mercapto-2-(R,S)-methylpropanoyl)-4,5,6,7-tetrahydro-thieno-[3,2-c]pyridin-4-(R,S)-carbonsäure;

D. 1-[3-(1-Phenyl-2-nitro-äthylthio)-2-R-methylpropanoyl]-L-prolin;

E. N-/N-1-(S-Ethoxycarbonyl-3-phenylpropyl)-2-S-alanyl7-4,5,6,7-tetrahydro-thieno/2,3-c7pyridin-7-R-carbonsäure; und

F. N-(3-Acetylthio-2-(S-Methylpropanoyl)-4,5,6,7-tetrahydrothieno-/3,2-c7-pyridin-4-R,S-carbonsäure;

und ihre pharmazeutischen Ester, Salze und Amide.

Ester und Salze dieser Verbindungen sind beispielsweise

G. 1-(3-Mercapto-2-D-methylpropanoyl)-L-prolin-pivaloyloxymethylester und

H. 1-{N-/(S)-1-Carboxy-3-phenylpropyl7-L-alanyl}-L-prolin-1'-ethylester maleat.

## Pharmakologische Untersuchungen

Die mucoprotektive Wirkung auf den Gastrointestinaltrakt verschiedener Verbindungen wurde im Vergleich zu dem bekannten Antiulcusmittel Carbenoxolone an weiblichen Ratten getestet, die für 24 Stunden vor Beginn des Tests fasteten. Diese Tiere wurden den folgenden Tests unterworfen:

1. Durch Ethanol verursachte Magengeschwüre

   Gruppen von 10 Ratten erhielten abgestufte Dosen der zu untersuchenden Verbindungen oral mittels Schlundsonde; die Verbindungen waren in 0,2 % Methocel suspendiert. Nach 30 Minuten erhielten die Tiere 0,7 ml absolutes Ethanol. 30 Minuten später wurden die Tiere getötet, der Magen entfernt, entlang der großen Curvatur geöffnet und von einem neutralen Beobachter untersucht.

Einem Magengeschwür entsprechende Schädigungen wurden auch nach ihrer Schwere beurteilt, aber ihre Häufigkeit (in %) diente als Basis für die Berechnung des prozentualen Schutzes. Die $ED_{50}$ ist diejenige Dosis einer Verbindung, die 50 % der Tiere schützt; sie wird ausgedrückt durch die Relation Schutz:Dosis.

Tabelle 1

| Verbindung | mg/kg p.o. | % Schutz (% Tier ohne Magengeschwüre) | $ED_{50}$ mg/kg |
|---|---|---|---|
| Captopril | 30 | 5 | |
| | 55 | 30 | 74,4 |
| | 100 | 70 | |
| G | 3 | 30 | |
| | 10 | 50 | 4,0 |
| | 30 | 40 | |
| C | ___ | ___ | 6 |
| D | ___ | ___ | 0,6 |
| E | ___ | ___ | 12 |
| F | ___ | ___ | 3 |
| Carbenoxolone | 30 | 10 | 89 |
| | 100 | 60 | |
| | 300 | 90 | |

2. Durch Natronlauge verursachte Magengeschwüre

Der Test verlief in gleicher Weise wie der unter 1. beschriebene. Anstelle von Ethanol wurde 0,2 N Natronlauge als Ulcus verursachendes Agens gewählt und die Tiere 60 Minuten nach dessen Gabe getötet. Die $ED_{50}$ wurde, wie oben angegeben, bestimmt.

Tabelle 2

| Verbindung | Dosis mg/kg p.o. | %Schutz-wirkung | $ED_{50}$ (mg/kg) |
|---|---|---|---|
| 1-(3-Mercapto-2-D-methylpropanoyl)-L-prolin pivaloyloxy-methylester | 3 | 10 | 11,7 |
| | 10 | 39 | |
| | 17 | 70 | |
| | 30 | 80 | |
| Carbenoxolone | 100 | 33 | 132 |
| | 170 | 70 | |
| | 300 | 80 | |

3. Durch Ethanol verursachte Magengeschwüre bei Ratten, die mit Indomethacin und Acetylsalicylsäure vorbehandelt worden waren

Es ist bekannt, daß Prostaglandine einen cytoprotektiven Effekt haben. Weiter ist bekannt, daß Prostaglandine eine antiulcerogene Wirkung haben. Durch eine Hemmung der Prostaglandin-Biosynthese, z.B. durch Acetylsalicylsäure oder Indomethacin, wird die antiulceregene Wirkung unterdrückt.

Gruppen von je 10 Ratten wurden entweder mit Indomethacin (3 mg/kg s.c.) oder Acetylsalicylsäure (10 mg/kg) vorbehandelt und nach 30 bis 60 Minuten die Testsubstanz verabreicht. Dann wurde ein durch Ethanol verursachtes Magengeschwür gesetzt und, wie unter 1. beschrieben, weitergearbeitet.

Tabelle 3

| Unterdrückung der Prostalglandin-Synthese: (30 Minuten vor der Ethanolgabe) Vorbehandlung | Wirkung von 1-(3-Mercapto-2-D-methylpropanoyl)-L-prolin-pivaloyloxy-methylester (30 mg/kg p.o.) % Schutzwirkung |
|---|---|
| Salzlösung (Kontrolle) | 100 |
| Indomethacin 3 mg/kg S.E. | 40 |
| Acetylsalicylsäure 10 mg/kg p.o. | 10 |

Gruppen von jeweils 10 Ratten. Indomethacin: 30 Minuten vor Ethanolgabe Acetylsalicylsäure: 60 Minuten vor Ethanolgabe

Die für die neue Indikation anwendbaren Verbindungen können warmblütigen Tieren vorzugsweise enteral verabreicht werden. Die Verbindungen liegen hierbei als aktive Bestandteile in üblichen Darreichungsformen vor, z.B. in Zusammensetzungen, die im wesentlichen aus einem inerten pharmazeutischen Träger und einer effektiven Dosis des Wirkstoffs bestehen, wie z.B. Tabletten, Dragees, Kapseln, Pulver, Lösungen, Suspensionen, Emulsionen oder Sirupe. Eine wirksame Dosis der erfindungsgemäßen Verbindungen liegt bei oraler Anwendung zwischen 5 und 100, vorzugsweise zwischen 10 und 50 mg/Dosis.

Nachfolgend werden Beispiele für einige pharmazeutische Zusammensetzungen unter Verwendung einer oder mehrerer Verbindungen der Struktur I als aktiver Bestandteil angegeben. Falls nicht ausdrücklich anders bezeichnet, handelt es sich bei den Teilen um Gewichtsteile.

## Pharmazeutische Anwendungsbeispiele

a) Dragees

1 Drageekern enthält:

| | |
|---|---|
| 1-(3-Mercapto-2-D-methylpropanoyl)-L-prolin-pivaloyloxymethylester | 25,0 mg |
| Milchzucker | 60,0 mg |
| Maisstärke | 35,0 mg |
| Gelatine | 3,0 mg |
| Magnesiumstearat | 2,0 mg |
| | 125,0 mg |

Herstellung:

Die Mischung der Wirksubstanz mit Milchzucker und Maisstärke wird mit einer 10%igen wäßrigen Gelatinelösung durch ein Sieb mit 1 mm Maschenweite granuliert, bei 40°C getrocknet und nochmals durch ein Sieb gerieben. Das so erhaltene Granulat wird mit Magnesiumstearat gemischt und verpreßt. Die so erhaltenen Kerne werden in üblicher Weise mit einer Hülle überzogen, die mit Hilfe einer wäßrigen Suspension von Zucker, Titandioxyd, Talkum und Gummi arabicum aufgebracht wird. Die fertigen Dragees werden mit Bienenwachs poliert.

b) Tabletten

| | |
|---|---|
| 1-(3-Mercapto-2-methylpropanoyl)-L-prolin-pivaloyloxymethylester | 50,0 mg |
| Milchzucker | 70,0 mg |
| Maisstärke | 50,0 mg |
| lösliche Stärke | 7,0 mg |
| Magnesiumstearat | 3,0 mg |
| | 180,0 mg |

Herstellung:

Wirkstoff und Magnesiumstearat werden mit einer wäßrigen Lösung der löslichen Stärke granuliert, das Granulat getrocknet und innig mit Milchzucker und Maisstärke vermischt. Das Gemisch wird sodann zu Tabletten von 180 mg Gewicht verpreßt, die je 50 mg Wirkstoff enthalten.

c) Kapseln

| | |
|---|---|
| 1-(3-Mercapto-2-D-methylpropanoyl)-L-prolin-pivaloyloxymethylester | 30,0 mg |
| Milchzucker | 200,0 mg |
| Maisstärke | 40,0 mg |
| Talk | 10,0 mg |
| | 280,0 mg |

Herstellung:

Wirkstoff, Milchzucker und Maisstärke werden zunächst in einem Mischer und dann in einer Zerkleinerungsmaschine vermengt. Das Gemisch wird nochmals in den Mischer gegeben, gründlich mit

dem Talk vermengt und maschinell in Hartgelatinekapseln abgefüllt.

Die Herstellung der für die neue Verwendung beanspruchten Verbindungen ist, wie bereits ausgeführt, bekannt. Nachfolgend wird beispielsweise die Herstellung eines der bereits bekannten ACE-Hemmer beschrieben:

Herstellungsbeispiel

1-(3-Mercapto-2-D-methylpropanoyl)-L-prolin-pivaloyloxy-methylester

Zu einer Suspension von 2 g (6,2 mMol) Quecksilber-II-acetat in 30 ml absolutem Ethanol werden bei 0°C unter Rühren 3,5 g (6,2 mMol) 1-(3-Tritylthio-2-D-methylpropanoyl)-L-prolin-pivaloyloxy-methylester in 30 ml absolutem Ethanol gegeben. Die gelbliche Lösung wird langsam auf Zimmertemperatur gebracht und bei dieser Temperatur 75 Minuten weitergerührt. Nach Abziehen des Ethanols im Vakuum wird der sirupöse Rückstand in Ether gelöst und mit Petrolether ausgefällt. Das Flüssigkeitsgemisch wird abdekantiert und der Rückstand zweimal mit Petrolether verrieben, wobei das anfänglich schmierige Produkt allmählich fest wird. Der Rückstand wird in 50 ml absolutem Ethanol gelöst und zwei Stunden lang unter Rühren $H_2S$ eingeleitet. Vom ausgefallenen Quecksilbersulfid wird abfiltriert und das Ethanol im Vakuum abdestilliert.
Man erhält das Endprodukt in einer Ausbeute von 1,55 g (76,6 % d. Th.) als Öl, das allmählich zu einer wachsweichen kristallinen Masse erstarrt.
Dünnschichtchromatographie auf Silicagel zeigt, daß das Produkt rein ist:
Rf = 0,558 (Essigester)
Rf = 0,63 (Benzol - Aceton 1:1)

Das Mercapto-Gruppenreagenz Nitroprussidnatrium zeigt im Dünnschichtchromatogramm eine positive Reaktion.

Die IR- und NMR-Daten bestätigen die Struktur der Titelverbindung.

Berechnet für $C_{15}H_{25}NO_5S$ (MG 331,43):

| | | | | | | | |
|---|---|---|---|---|---|---|---|
| C | 54,36 | H | 7,60 | N | 4,23 | S | 9,67 |

Gefunden:

| | | | | | | | |
|---|---|---|---|---|---|---|---|
| C | 54,64 | H | 7,45 | N | 4,09 | S | 9,55 |

$/\alpha/_D$: - 112,8 (C = 1, Ethanol)

## PATENTANSPRÜCHE

1. Pharmazeutische Zusammensetzungen mit mucoprotektiver Wirkung auf den Gastrointestinaltrakt, enthaltend Verbindungen der Struktur

A-B-P     I.

in der A für geradkettigen oder verzweigten Alkyl- beziehungsweise Acylrest, der substituiert sein kann durch SH, COOH oder COO-Alkyl und der gegebenenfalls unterbrochen sein kann, durch eine CO-Gruppe oder ein Schwefelatom; einen Phenylalkyl- beziehungsweise Phenylacylrest, wobei die Alkyl- beziehungsweise Acylkette geradkettigen oder verzweigt und ein- oder vielfältig substituiert sein kann, durch SH, S-Alkyl, S-Acyl, S-Alkylaminocarbonyl, COOH, COO-Alkyl oder $NO_2$ und gegebenenfalls unterbrochen sein kann, durch eine CO-Gruppe oder ein Schwefelatom, steht;

P für Carboxypyrrolidinyl oder Carboxypiperidinyl gegebenenfalls substituiert durch eine oder mehrere Hydroxy- oder Oxogruppe oder gegebenenfalls geminal substituiert durch Dialkoxy, Dithioalkoxy, Alkylendioxy oder Alkylendithio, und/oder gegebenenfalls substituiert durch/oder ankondensiert mit einem 5- oder 6-Ring--Heterocyclus wie Furan, Pyrrol, Thiophen, Benzofuran, Indol, Benzothiophen, Oxazol, Imidazol, Thiazol, Isoxazol, Pyrazol, Pyrrolidin, Tetrahydrofuran, Tetrahydrothiophen, Pyridin, Pyridazin, Chinolin, Isochinolin oder Piperidin, oder Thiazolidincarbonsäure steht;

und B für eine Einfachbindung, eine Aminosäure oder mehrere über Peptidbindung(en) miteinander verknüpften Aminosäuren, steht,

und falls das Molekül eine SH-Gruppe enthält, über eine -S-S-Bindung ein Doppelmolekül bilden kann;

oder ihre pharmazeutisch annehmbaren Salze, Ester oder Amide.

2. Pharmazeutische Zusammensetzungen mit mucoprotektiver Wirkung auf den Gastrointestinaltrakt nach Anspruch 1, worin A für einen 3-Mercapto-2-methyl-propionyl, 3-Acetylthio-2-methylpropionyl, 3-Mercaptopropionyl-, 3-Mercapto-butyryl-, 2-Methyl-4-ethoxycarbonyl-pentanoyl-, 3-Acetylthiobutyryl-, 3-Trimethylacetylthiobutyryl-, Hydroxycarbonyl-methyl-thiopropionyl-, 3-Phenyl-2-mercaptopropionyl-, 3-Phenyl-2-methyl-mercaptopropionyl-, 3-Phenyl-2-benzoylmercaptopropionyl-, 3-Phenyl-2-trimethyl-acetylmercapto-propionyl-, 3-Phenyl-2-ethylamino-carbonyl-mercaptopropionyl-, 1-Ethoxycarbonyl-3-phenyl-propyl-, 1-Hydroxycarbonyl-3-phenyl-propyl- oder 3-(2-Nitro-1-phenyl-ethylthio)-2-methylpropionyl-rest, steht.

3. Pharmazeutische Zusammensetzungen gemäß Anspruch 2, worin A für 3-Acetylthio-2-methylpropionyl, 3-Mercapto-2-methylpropionyl, 3-Phenyl-2-mercaptopropionyl, 1-Ethoxy-3-phenylpropyl, 1-Hydroxycarbonyl-3-phenylpropyl oder 3-(2-Nitro-1-phenylethylthio)-2-methyl-propionyl, steht.

4. Pharmazeutische Zusammensetzung gemäß Anspruch 1, 2 oder 3, worin B für eine Einfachbindung oder einen Aminosäurerest, der sich von Glycin, Alanin, Serin oder Lysin ableitet, steht.

5. Pharmazeutische Zusammensetzung gemäß einer der Ansprüche 1 bis 4, worin P für 2-Carboxypyrrolidinyl, 4,5,6,7-Tetrahydrothieno-/3,2-c/piperidinyl-4-carbonsäure oder 4,5,6,7-tetrahydrothieno-/2,3-c/piperidinyl-7-carbonsäure, steht.

6. Pharmazeutische Zusammensetzungen mit mucoprotektiver Wirkung auf den Gastrointestinaltrakt enthaltend
1-(3-Mercapto-2-D-methyl-propanoyl)-prolin

1- N-/(S)-1-Carboxy-3-phenylpropyl/-L-alanyl -L-prolin-1'-ethylester,

1-(3-Mercapto-2(R,S)-methylpropanoyl-4,5,6,7-tetrahydro-thieno-/3,2-c/pyridin-4-(R,S)-carbonsäure,

1-/3-(1-Phenyl-2-nitro-ethylthio)-2-R-methylpropanoyl7-L-prolin,
N-/N-1-S-(Ethoxycarbonyl-3-phenylpropyl)-2-S-alanyl7-4,5,6,7-tetrahydro-thieno/2,3-c7pyridin-7-R-carbonsäure, oder
N-(3-Acetylthio-2-(S-methylpropanoyl)-4,5,6,7-tetrahydrothieno-/3,2-c7pyridin-4-R,S-carbonsäure,

oder ihrer Salze, Ester oder Amide.

7. Pharmazeutische Zusammensetzungen gemäß Anspruch 6, gekennzeichnet durch einen Gehalt von
1-(3-Mercapto-2-D-methylpropanoyl)-prolin-pivaloyloxy-methyleser oder

1-{N-/(S)-1-Carboxy-3-phenylpropyl7-L-alanyl}-L-prolin-1'-ethylester maleat.

8. Verwendung einer oder mehrerer Verbindungen der Formel I gemäß Anspruch 1 bis 7, zur Behandlung von Schleimhauterkrankungen des Magen-Darmtraktes.

9. Verwendung einer Verbindung der Formel I nach einem der Ansprüche 1 bis 7, zur Herstellung eines Arzneimittels zur Behandlung von Schleimhautdefekten des Magen-Darmtraktes.